(19)

Europäisches
Patentamt
European
Patent Office
Office européen
des brevets

(11) **EP 4 170 661 A1**

(12) **EUROPEAN PATENT APPLICATION**

(43) Date of publication:
**26.04.2023 Bulletin 2023/17**

(21) Application number: **21306458.7**

(22) Date of filing: **19.10.2021**

(51) International Patent Classification (IPC):
**G16B 20/40** (2019.01)    **G16B 40/10** (2019.01)
**G16B 20/30** (2019.01)

(52) Cooperative Patent Classification (CPC):
**G16B 20/40; G16B 20/30; G16B 40/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Institut National de la Santé et de la
Recherche Médicale (INSERM)
75013 Paris (FR)**

• **Université de Lorraine
54000 Nancy (FR)**

(72) Inventors:
• **OUSSALAH, Abderrahim
54850 Messein (FR)**
• **GUEANT, Jean-Louis
54000 Nancy (FR)**

(74) Representative: **Plasseraud IP
66, rue de la Chaussée d'Antin
75440 Paris Cedex 09 (FR)**

(54) **METHYLATION PROFILE ANALYSIS USING SMOOTHING METHOD**

(57)    The inventors showed that by applying the smoothing method, they optimized the signal-to-noise ratio, which allowed highlighting potentially relevant epigenomic signatures. The optimization of the signal-to-noise ratio resulted from a downward shift (shift to the left) of the distribution of the —Log10(P-values) at the genome-wide level, allowing background noise to be reduced while maintaining the strength of the signal. Accordingly, the present invention relates to a smoothing method for analyzing methylation profile of DNA samples of a population and a method for identifying a DNA methylation signature associated with a pathological or physiological state. The present invention also relates to the use of a DNA methylation signature for diagnosing hepatocellular carcinoma in a subject.

**No smoothing (radius = 0)**

Unsmoothed -Log10(P-value)

**Smoothing radius = $r$**

$k$-3  $k$-2  $k$-1  $k$  $k$+1  $k$+2  $k$+3

$$\text{Smoothed}(Pk, w) = \sum_{n=k-w}^{n=k+w} (-Log10[P - value]) \div (2w + 1)$$

**FIG. 1**

Processed by Luminess, 75001 PARIS (FR)

## Description

### TECHNOLOGY FIELD OF THE INVENTION

The present invention relates to a smoothing method for analyzing the methylation profile of DNA samples of a population and a method for identifying a DNA methylation signature associated with a pathological or physiological state.

### BACKGROUND OF THE INVENTION

The advent of genome-wide methods for assessing the epigenome landscape, notably the DNA methylome, has revolutionized the understanding of changes and alterations of epigenetic architecture in complex human traits and human diseases', respectively (Michels, K. B. et al. Nat Methods 10, 949-955, (2013)). Epigenome-wide association studies (EWAS) use microarrays to analyze the methylation of CpG dinucleotides (also called CpG probes) at the genome-wide level. The ability of EWAS to translate into biologically meaningful results relies on the detection of epigenomic signatures with a high level of statistical certainty. However, EWAS may be prone to bias, potentially leading to spurious associations, notably in case series with small sample sizes (Michels, K. B. et al. Nat Methods 10, 949-955, (2013); van Iterson, M., et al. Genome Biol 18, 19, (2017)).

Several analytical methods have been developed to identify epigenomic signatures in EWAS (Mallik, S. et al. Brief Bioinform 20, 2224-2235, (2019)). Among them, the supervised approaches based on differentially methylated region (DMR) calling and the intelligent classifiers represent the most widely used (Pedersen, B. S., et al. Bioinformatics 28, 2986-2988, (2012); Jaffe, A. E. et al. Int J Epidemiol 41, 200-209, (2012) ; Zou, J., et al. Nat Methods 11, 309-311, (2014) ; Li, H., Hong, et al. Gene 555, 203-207, (2015); Lehne, B. et al. Genome Biol 16, 37, (2015); Fan, S. & Chi, W. Brief Funct Genomics 15, 432-442, (2016); Chen, J. et al. BMC Genomics 18, 413, (2017); Heiss, J. A. et al. Epigenomics 9, 13-20, (2017); Rahmani, E. et al. Genome Biol 19, 141, (2018); Zheng, S. C., et al. Nat Methods 15, 1059-1066, (2018); Saffari, A. et al. Genet Epidemiol 42, 20-33, (2018); Srivastava, A., et al. BMC Bioinformatics 20, 253, (2019)). The DMR calling methods are based on CpG annotations and use conventional statistical approaches (e.g., Student's $t$-test (Bibikova, M. et al. Genome Res 16, 1075-1083, (2006)) or more elaborated methods (e.g., beta-binomial regression fit (Hebestreit, K., Dugas, M. & Klein, H. U. Bioinformatics 29, 1647-1653, (2013) or Shannon entropy (Xie, H. et al. Nucleic Acids Res 39, 4099-4108, (2011)). All these methods and algorithms are well detailed in the review by Fan *et al.* (Fan, S. & Chi, W. Brief Funct Genomics 15, 432-442, (2016)), and as suggested by the authors, the supervised methods are in line with the classical paradigms of study power and multiple testing correction, and it remains a need to improve the statistical power for detecting the weak differences.

The smoothing method utilizes the concepts of signal processing theory to mirror the biological reality of the DNA methylome architecture. Formerly, several smoothing methods have been developed as mathematical models for signal de-noising, notably in engineering, spectrometry, and image processing (Angenent, S., Pichon, E. & Tannenbaum, A. Bulletin of the American mathematical society 43, 365-396 (2006); Kuan, D. T., et al., 165-177 (1985); O'Haver, T. & Begley, Analytical Chemistry 53, 1876-1878 (1981); Czanner, G. et al. Proc Natl Acad Sci U S A, 112, 7141-7146, (2015); Qi, J. et al. Nat Commun, 9, 1848, (2018)).

Jaffe et al. reported a 'bump hunting' approach for DNA methylome analysis that relies on the co-methylation pattern of CpG probes (Jaffe, A. E. et al. Int J Epidemiol 41, 200-209, (2012)). They modeled the methylation level of CpG probes as a smooth function of genomic position and they implemented a multistep approach to identify differentially methylated regions based on a logit-transformation of methylation levels of CpG probes depending on the outcome of interest, an estimation of smoothed beta-values, and statistical testing to assess the statistical uncertainty to each estimated differentially methylated region. However, this method requires statistical power and adjustment to multiple testing constraints, and it remains a need to improve the method for analyzing the DNA methylome with a high degree of accuracy.

### SUMMARY OF THE INVENTION

In this way, the smoothing method proposed in the present application in the setting of EWAS analyses overcomes both statistical power and adjustment to multiple testing constraints since it handles nominal P-values to reconstruct an updated epi-Manhattan plot after signal processing.

In a landmark study, Eckhardt *et al.* used bisulfite DNA sequencing to generate high-resolution methylation profiles of human chromosomes 6, 20, and 22, and showed that over 90% of CpG sites within 50 bases had the same methylation status (Eckhardt, F. et al. Nat Genet 38, 1378-1385, (2006); Bell, J. T. et al. Genome Biol 12, R10, (2011); Bibikova, M. et al. Genomics 98, 288-295, (2011)). Based on this concept of the co-methylation pattern of CpG dinucleotides, notably within CpG islands, the inventors proposed the smoothing method at the genome-wide level through a sliding window approach to calculate and visualize data from EWAS in order to decipher the most informative epigenetic alterations with a high degree of accuracy. The inventors have illustrated the smoothing method by performing several EWAS from various settings, including two monogenic epigenetic diseases *(epi-cblC* and primary constitutional *MLH1* epimutation) and an EWAS on epigenetic predictors of aging. To assess the magnitude of signal enhancement at the top significant loci and evaluate the benefit provided by the smoothing method, the inventors used several metrics, including the signal-to-noise ratio (SNR), variance reduction, and variance ratio (VR), that demonstrated the method's efficiency. Importantly, by applying the smoothing method, only the genomic regions carrying an aberrant epimutation related to monogenic disorders (e.g., *MMACHC* promoter CpG island and *MLH1* CpG island) reached a top significant association without any other spurious association at the genome-wide level. Using the smoothing method in an EWAS of aging epigenetic predictors, showed that it remained efficient even after applying an 80% reduction of the original sample size. The present invention relates to a computer-implemented method for analyzing methylation profiles of DNA samples of a population, where each methylation profile comprises a methylation level of cytosine of a plurality of CpG sites of determined genomic DNA positions, the method comprising: i) performing a statistical test on said methylation profiles of the population, and obtaining a nominal p-value for a plurality of said CpG sites, ii) for at least one CpG site, computing a transformed p-value taking into account the nominal p-value for the considered CpG site and its genomic position. In a particular embodiment, the computation of the transformed p-values takes into account the nominal p-value obtained for the considered CpG site and the nominal p-values obtained for CpG sites having neighboring DNA positions. In a preferred embodiment, the computation of the transformed p-values takes into account the nominal p-value obtained for the considered CpG site and the nominal p-values obtained for CpG sites belonging to respectively a same CpG-rich region to which belongs the considered CpG site. In a more preferred embodiment, computing a transformed p-value comprises computing, for a given CpG site, a mean of log-transformed nominal p-values of the CpG sites comprised in a sliding window centered on the given CpG site and including 2w+1 CpG sites where w is a positive integer designating the width of the sliding window, preferably computing a transformed p-value comprises computing, for a given CpG site a value defined by the following equation:

$$Smoothed(Pk,w) = \sum_{n=k-w}^{n=k+w} (-Log10[P - value(Pn)]) \div (2w + 1)$$

where Smoothed(Pk,w) is the transformed p-value for the CpG site of location Pk within the methylation profile, w is the width of the sliding window, and P-value(Pn) is the nominal p-value of the CpG site of location Pn. In a preferred embodiment, the method further comprises computing a threshold on the transformed p-values and identifying at least one CpG site exhibiting a transformed p-value higher than said threshold. In a more preferred embodiment, said method further comprises displaying a Manhattan plot of the transformed p-values. In a preferred embodiment, said methylation level is the ratio of methylated cytosine and the sum of methylated and unmethylated cytosines.

The method according to the present invention may comprise prior to step i) the step of determining methylation level in said DNA samples of a population, preferably wherein the step of determining said methylation level comprises a bisulfite pre-treatment of the DNA samples which converts unmethylated cytosines into uracils, more preferably wherein the step of determining said methylation level comprises applying DNA samples on microarray chips of probes.

In another aspect, the present invention relates to a method for identifying a methylation signature associated with a physiological or pathological state comprising implementing the method as described above wherein said population comprises at least test subjects having said physiological or pathological state, preferably wherein the population further comprises control subjects not having said pathological or physiological state.

**FIGURE LEGENDS**

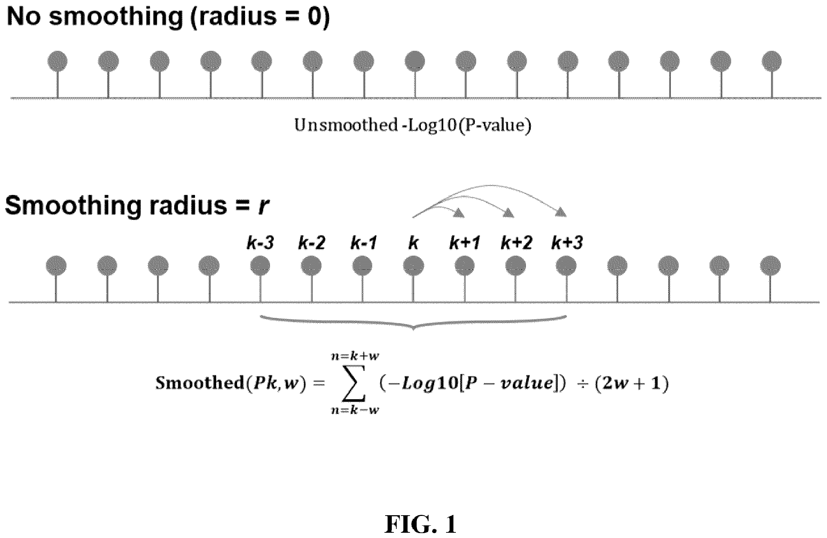

**Figure 1. Conceptual representation of the smoothing method for calculating and visualizing data from epigenome-wide association studies.** The smoothing method relies on the DNA co-methylation pattern, which is the correlation between adjacent CpG sites. The smoothing method consists of the conversion of the nominal -Log10(*P*-value) obtained for each CpG probe at the genome-wide level to smoothed P-values (Smoothed[$P_{k,w}$]) using a pre-specified window width value (w). A detailed description of the smoothing method and the corresponding statistical formula are reported in Online Methods.

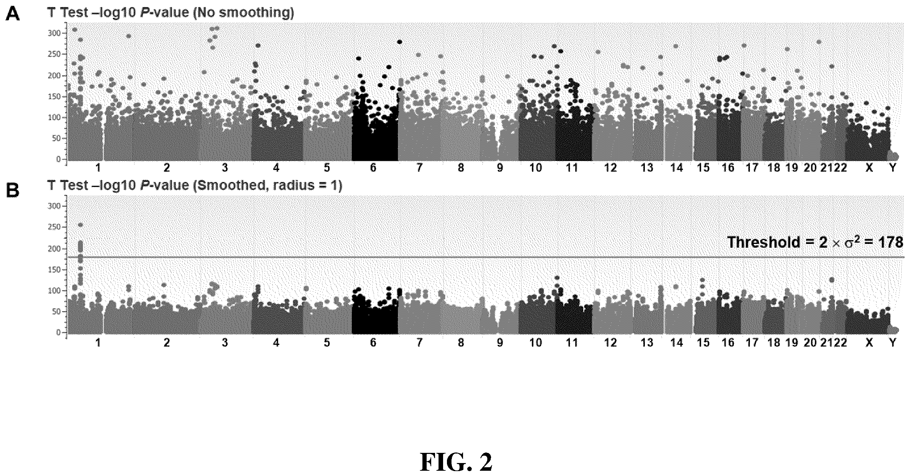

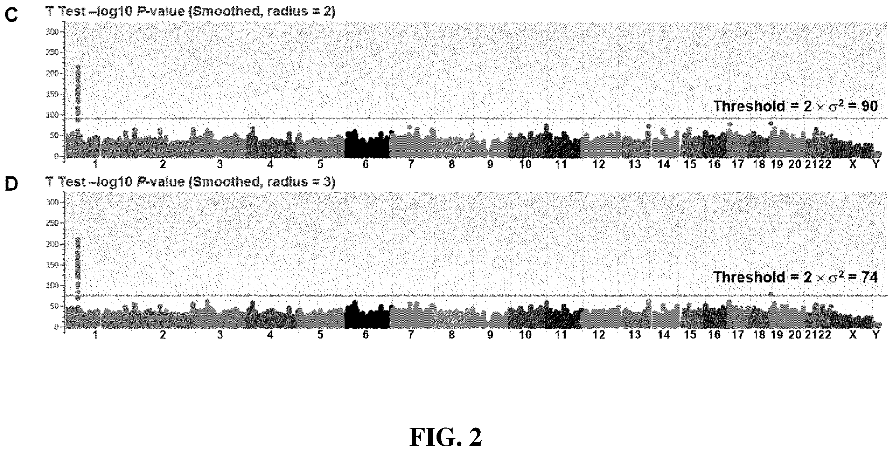

**Figure 2. Epi-Manhattan plots reporting the epigenome-wide association study that compared the DNA**

**methylome profiles of 16 subjects with** *MMACHC* **promoter epimutation (CpG island CpG:33) with control subjects from the MARTHA population.** Panel (A) reports the epi-Manhattan plot using untransformed -Log10(*P*-values). Panels (B), (C), and (D) report the epi-Manhattan plot after applying a smoothing transformation at the genome-wide level using a window width of 1, 2, and 3, respectively. The red line represents the -Log10(*P*-value) threshold after the smoothing transformation ($\sigma^2$ corresponds to the variance).

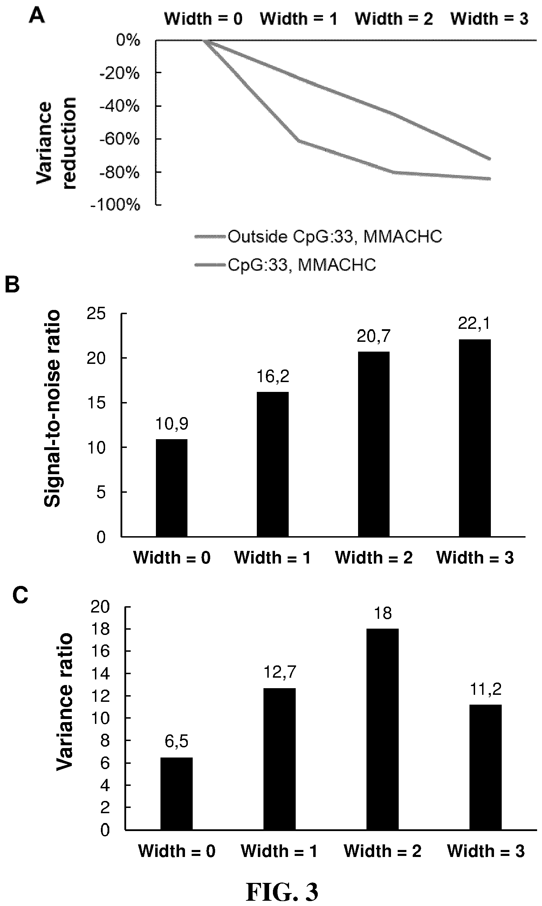

**Figure 3. Variance reduction (A), signal-to-noise ratio (B), and variance ratio (C) as metrics to estimate the magnitude of signal enhancement at the** *MMACHC* **locus (CpG island CpG:33).** The variance reduction was calculated by considering the variance obtained from untransformed -Log10(*P*-values) as a reference value. The signal-to-noise ratio (SNR) was defined as the ratio of the mean of smoothed -Log10(*P*-values) (Smoothed[$P_{k,w}$]) of the CpG probes located in the analyzed top significant locus on the standard deviation of Smoothed($P_{k,w}$) of all the CpG probes located outside the analyzed top significant locus at the genome-wide level, using the same window width value (w).

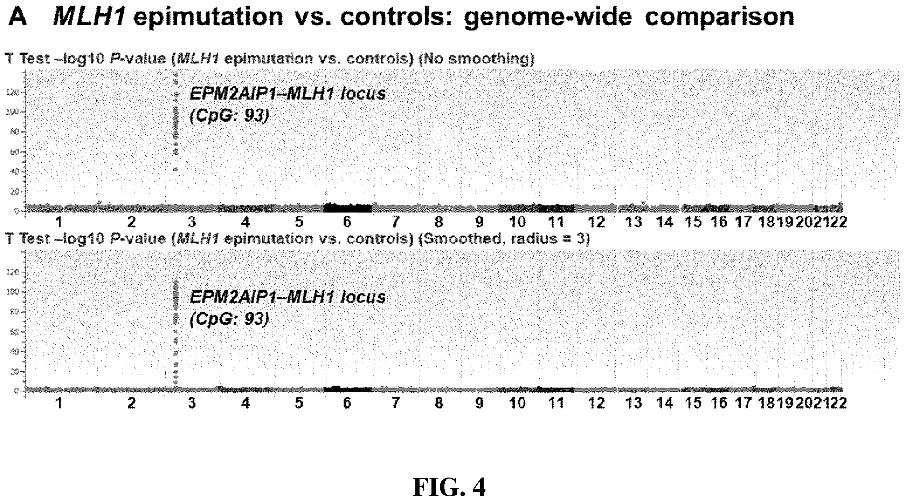

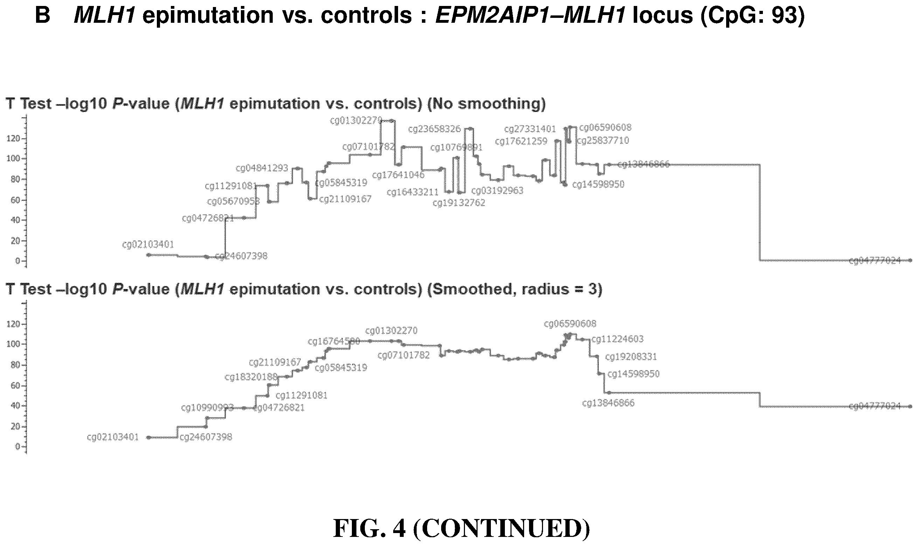

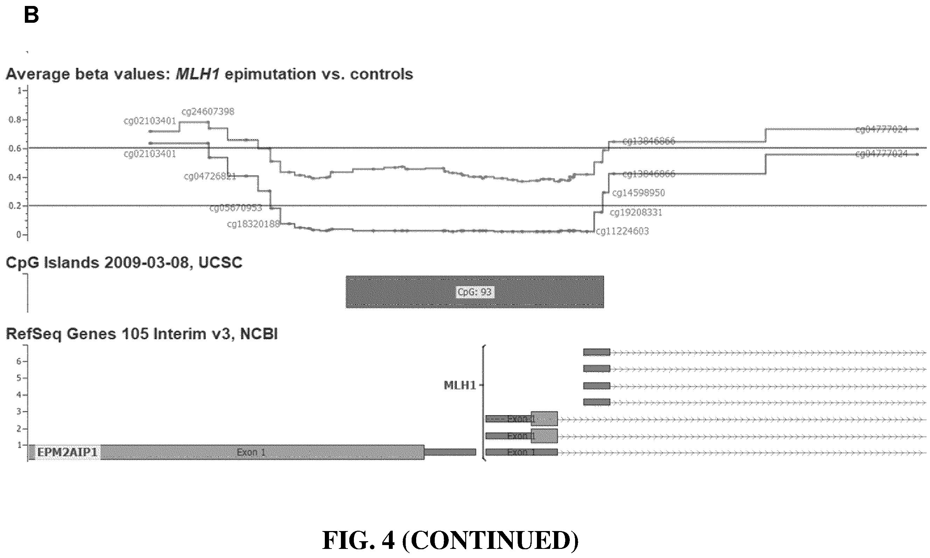

**Figure 4. Epi-Manhattan plot reporting the epigenome-wide association study that compared the DNA methylome profiles of 12 patients with** *MLH1* **epimutation with that of 102 patients without** *MLH1* **epimutation.** The top and bottom of the (A) panel report the epi-Manhattan plot using untransformed -Log10(*P*-values) and transformed - Log10(*P*-values) after applying a smoothing transformation at the genome-wide level using a window width of 3, respectively. Panel (B) shows the zoomed view on the *EPM2AIP1-MLH1* bidirectional promoter CpG island (CpG: 93). From top to bottom, the following items are shown: untransformed -Log10(*P*-values), transformed - Log10(*P*-values) using a smoothing width of 3, average beta-values in cases with *EPM2AIP1-MLH1* epimutation (red line), and average beta-values in patients without *EPM2AIP1-MLH1* epimutation (blue line). The position of the CpG island and the genomic context are shown at the bottom of the (B) panel.

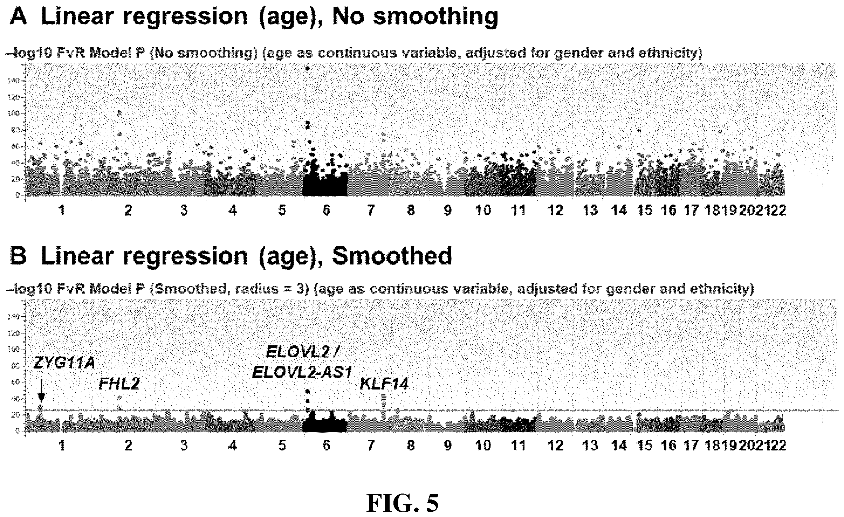

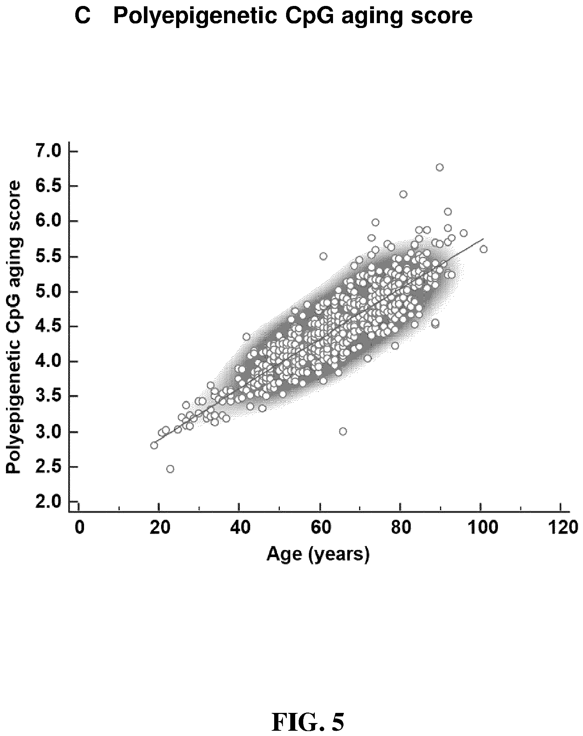

**Figure 5. Epi-Manhattan plot reporting the epigenome-wide association study on 656 subjects that assessed potential epigenetic predictors associated with age after adjusting for gender and ethnicity**. Panel (A) reports the epi-Manhattan plot using untransformed -Log10(*P*-values). Panel (B) reports the epi-Manhattan plot after applying a smoothing transformation at the genome-wide level using a window width of 3. The top significant loci in association with age are indicated in panel (B). (C) Correlation between the 'Polyepigenetic CpG aging score' and patients' age expressed in years. The 'Polyepigenetic CpG aging score' was derived from the 15 top CpG probes in the four loci associated with age.

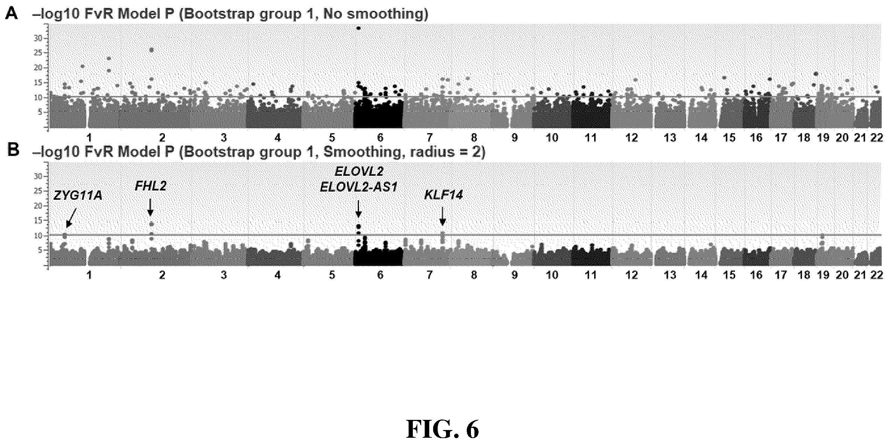

Figure 6. Epi-Manhattan plot reporting a sensitivity analysis on a randomly selected subsample representing 20% of the total population. Panels (A) and (B) report the epi-Manhattan plot using untransformed -Log10(*P*-values) and after applying a smoothing transformation at the genome-wide level using a window width of 2.

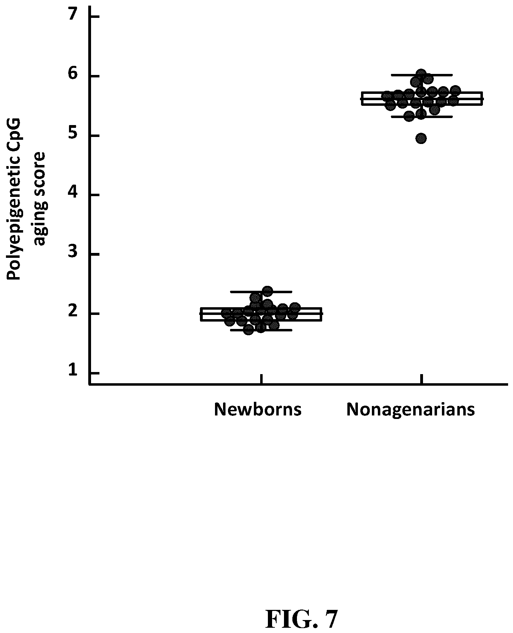

**Figure 7. Box-plot reporting the median value of the 'Polyepigenetic CpG aging score' in an independent replication study of blood methylome profiling in newborns and nonagenarians**. The median value of the 'Polyepigenetic CpG aging score' was 2.00 (IQR, 1.89-2.08) and 5.60 (IQR, 5.52-5.72) in newborns and nonagenarians, respectively (P = $3.40\times10^{-8}$).

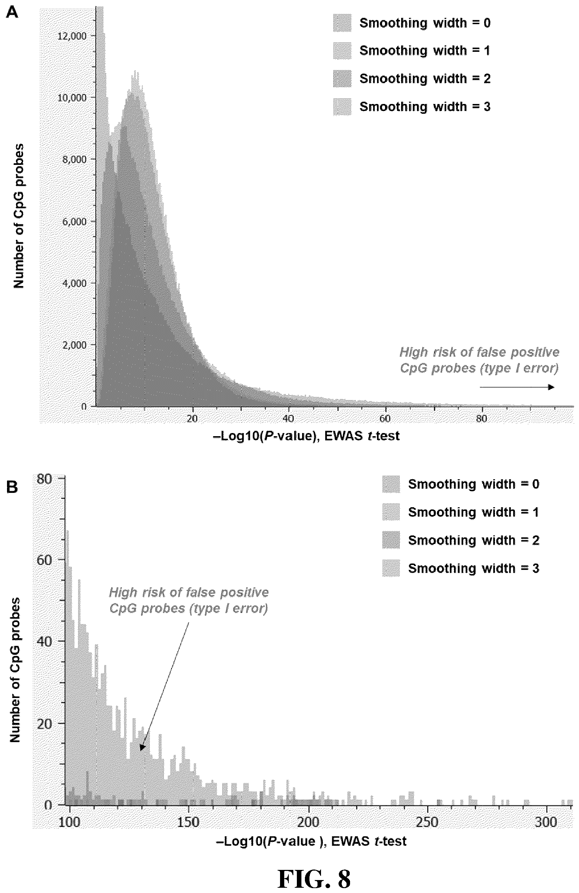

**Figure 8. Density distribution of CpG probes according to their untransformed - Log10(*P*-values) and after applying a smoothing transformation of -Log10(*P*-values) (data reported from the EWAS in Example #1)**. Panel (A) reports the CpG probes with a -Log10(*P*-value) ranging between 0 and 100; Panel (B) reports CpG probes with a -Log10(*P*-value) ranging between 100 310. In panel (B), the CpG probes with high untransformed -Log10(*P*-values) exhibit a high risk of false-positive association. After applying the smoothing transformation, the number of CpG probes with smoothed -Log10(*P*-values) >100 (width = 1, 2, or 3) is dramatically decreased.

## DETAILED DESCRIPTION OF THE INVENTION

### Definitions

As used herein, the term "nucleic acid sequence" refers to a single- or double-stranded nucleic acid. Said nucleic acid sequence can be DNA or RNA. In preferred embodiments, the "nucleic acid sequence" is a double-stranded DNA.

The term "DNA methylation profile" or "methylation profile" refers to a pattern of methylation level of cytosine of a set of CpG sites of determined genomic DNA positions in DNA sample that generally occurs as a result of a biological process, e.g. pathological or physiological state.

As used herein, the term "CpG site" or "CpG probe" refers to the position in DNA sequence where a cytosine and guanine are adjacent and the 3'oxygen of the cytosine is covalently attached to the 5'Phosphate of the guanine. Cytosines in CpG dinucleotides can be methylated to form 5-methylcytosine.

As used herein, the term "methylation state" or "methylation level" as used herein describes the state of methylation of a CpG site, thus refers to the presence or absence of 5-methylcytosine at one cpG site within DNA. When none of the DNA of an individual is methylated at one given CpG site, the position is 0% methylated. When all the DNA of the individual is methylated at that given CpG site, the position is 100% methylated. When only a portion, e.g., 50%, 75%, or 80% of the DNA of the individual is methylated at said CpG site, then the CpG position is said to be 50%, 75%, 80% methylated, respectively.

The term "methylation level" reflects any relative or absolute amount of methylation of a CpG position. The terms "methylation" and "hypermethylation" are used herein interchangeably. When used in reference to a CpG position, they refer to the methylation status corresponding to an increased or decreased presence of 5-methylcytosine at a CpG site within the DNA sequence of a biological sample obtained from a population (e.g., patient) relative to the amount of 5-methylcytosine found at the CpG site within the same DNA position of a biological sample obtained from a control value (e.g., healthy individual).

In a particular embodiment, said methylation state at the CpG site is a beta-value that corresponds to the ratio of methylated cytosine and the sum of methylated and unmethylated cytosines at the CpG site.

The term "CpG-rich region" refers herein to a genomic DNA region of at least 200 bp that has more than 50% CpG content. In particular embodiment, said GpG rich region is CpG island, promoter region or gene body.

The term "subject" refers to both human and non-human animals. As used herein, the term "subject" denotes a mammal, such as a rodent, a feline, a canine, and a primate. Preferably, a subject according to the invention is a human.

The term "biological sample" or "sample" is generally obtained from a subject or from a population of subjects. A sample may be any biological tissue or fluid with which the methylation status of CpG sites of the present disclosure may be assayed. Frequently, a sample will be a "clinical sample" (i.e., a sample obtained or derived from a patient to be tested). The sample may also be an archival sample with a known diagnosis, treatment, and/or outcome history. Examples of biological samples suitable for use in the practice of the present disclosure include, but are not limited to, bodily fluids, e.g., blood samples (e.g., blood smears), and cerebrospinal fluid, brain tissue samples or bone marrow tissue samples such as tissue or fine needle biopsy samples. Biological samples may also include sections of tissues, such as frozen sections taken for histological purposes. The term "biological sample" also encompasses any material derived by processing a biological sample. Derived materials include, but are not limited to, cells (or their progeny) isolated from the sample, as well as nucleic acid molecules (DNA and/or RNA) extracted from the sample. The biological sample may be circulating free DNA (cfDNA), which corresponds to degraded DNA fragments released to the blood plasma. cfDNA can be derived from healthy or tumor cells.

The method, according to the present disclosure, may include a step of determining the methylation level of cytosine of a plurality of CpG sites in determined DNA positions of a sample obtained from a population.

The determination of the methylation level may be performed using any method known in the art to be suitable for assessing the methylation of cytosine residues in DNA. Such methods are known in the art and have been described; and one skilled in the art will know how to select the most suitable method depending on the number of samples to be tested, the quantity of sample available, and the like.

For example, methylation can be measured by employing a restriction enzyme-based technology, which utilizes methylation-sensitive restriction endonucleases for the differentiation between methylated and unmethylated cytosines. Restriction enzyme-based technologies include, for example, restriction digest with methylation-sensitive restriction enzymes followed by nucleic acid sequencing (e.g., massively parallel or Next Generation sequencing), Southern blot analysis, real-time PCR, restriction landmark genomic scanning (RLGS) or differential methylation hybridization (DMH).

In some embodiments that employ methylation-sensitive restriction enzymes, a postdigest PCR amplification step is added wherein a set of two oligonucleotide primers, one on each side of the methylation-sensitive restriction site, is used to amplify the digested genomic DNA. PCR products are produced and detected for templates that were not restricted (e.g., due to the presence of a methylated restriction site) whereas PCR products are not produced where digestion of the subtended methylation-sensitive restriction enzyme site occurs. Techniques for restriction enzyme-based analysis of genomic methylation are well known in the art and include the following: differential methylation hybridization (DMH) (Huang et al., 1999, Human Mol. Genet. 8, 459-70); Not I-based differential methylation hybridization (for example, WO02/086163A1); restriction landmark genomic scanning (RLGS) (Plass et al., 1999, Genomics 58:254-62); methylation-sensitive arbitrarily primed PCR (AP-PCR) (Gonzalgo et al., 1997, Cancer Res. 57: 594-599); methylated CpG site amplification (MCA) (Toyota et al., 1999, Cancer Res. 59: 2307-2312).

In a preferred embodiment, the methods rely on the prior treatment of DNA with sodium bisulfite. This treatment leads to the conversion of unmethylated cytosine to uracil, while methylated cytosine remains unchanged (Clark et al., Nucleic Acids Res., 1994, 22: 2990-2997). This change in the DNA sequence following bisulfite conversion can be detected using a variety of methods, including PCR amplification followed by DNA hybridization on a microarray of probes. The protocol described by Frommer et al. (PNAS USA, 1992, 89: 1827-183 1) has been widely used for sodium bisulfite treatment of DNA, and a variety of commercial kits are now available for this purpose.

Techniques for the analysis of bisulfite-treated DNA can employ methylation-sensitive primers for the analysis of CpG methylation status with isolated genomic DNA, for example, as described by Herman et al., 1996, Proc. Natl. Acad. Sci. USA 93:9821-9826, or U.S. Pat. Nos. 5,786,146 or 6,265,171). Methylation sensitive PCR (MSP) allows for the detection of a specific methylated CpG position within, for example, the regulatory region of a gene. The DNA of interest is treated such that methylated and unmethylated cytosines are differentially modified, for example, by bisulfite treatment, in a manner discernable by their hybridization behavior. PCR primers specific to each of the methylated and unmethylated states of the DNA are used in PCR amplification. Products of the amplification reaction are then detected, allowing for the deduction of the methylation status of the CpG position within the genomic DNA. Other methods for the analysis of bisulfite-treated DNA include methylation-sensitive single nucleotide primer extension (Ms-SNuPE) (see, for example, Gonzalgo & Jones, 1997; Nucleic Acids Res. 25:2529-253 1, or U.S. Patent 6,251,594), or the use of real-time PCR based methods, such as the art-recognized fluorescence-based real-time PCR technique MethyLight™ (see, for example, Eads et al., 1999; Cancer Res. 59:2302-2306, U.S. Pat. No. 6,331,393 or Heid et al., 1996, Genome Res. 6:986-994). It will be understood that a variety of methylation assay methods can be used for the determination of the methylation status of particular genomic CpG positions. Methods that employ bisulfite conversion include, for example, bisulfite sequencing, methylation-specific PCR, methylation-sensitive single nucleotide primer extension (Ms-SnuPE), MALDI mass spectrometry and methylation-specific oligonucleotide arrays, for example, as described in U.S. Pat. No. 7,61 1,869 or International Patent Application WO2004/05 1224).

In a preferred embodiment, the methylation level of a plurality of CpG sites in a sample is detected using an array or chip of probes. In such embodiments, a plurality of different probe molecules can be attached to a substrate or otherwise spatially distinguished in an array. Said probes are labeled, for example, with a fluorescent molecule, and the level of methylation can be determined by calculating the ratio of the fluorescence signals from the methylated versus unmethylated sites.

A variety of commercially available array-based products to detect methylation can be used, including, for example, the MethylationEPIC™ BeadChip™ (Illumina, Inc., San Diego, CA), which allows interrogation of over 850,000 methylation sites quantitatively across the human genome at single-nucleotide resolution.

**Analysis of methylation profile**

The present disclosure provides a method for analyzing methylation profiles of DNA samples of a population, said methylation profiles comprising methylation levels of a plurality of CpG of determined genomic DNA positions.

The analysis method is performed by a computer comprising processing means which can include, for instance, one or a plurality of processor(s), microprocessor(s), microcontroller(s), and memory storing code instructions which are executed by the computer to perform the method detailed below. In addition, the computer preferably comprises a display such as a screen for visualization of the output of the analysis.

The analysis method may comprise a preliminary step of acquiring methylation profiles of DNA samples of a population according to the determination method disclosed above. Alternatively, the analysis method may be performed on previously acquired methylation profiles which can be recovered from a storage database.

The population comprises a plurality of subjects. In embodiments where the analysis is performed in a context of a case-control study, the population may comprise at least one, and preferably a plurality of case subjects associated with a physiological or pathological state, and a plurality of control subjects not having said physiological or pathological state. In embodiments where the analysis is performed in a context of a cohort study, the population comprises a plurality of subjects associated with a continuous variable.

The method comprises performing a statistical test on the methylation profiles of the population in order to obtain a nominal p-value for a plurality of CpG sites. In embodiments, a nominal p-value is obtained for each CpG site for which the methylation profile of a subject of the population comprises a methylation level. The nominal p-value corresponds to a probability of obtaining a test results at least as extreme as the results actually observed, under the assumption that the test hypothesis ("null hypothesis" is correct). The computation of the nominal p-value thus depends on the statistical test which is implemented. It is considered that a p-value should be inferior to 0.05 to confirm a test hypothesis.

The statistical test may be selected among frequentist or Bayesian approaches. The statistical test may be selected among a plurality of statistical tests known to the skilled person, depending on the nature of the study.

In the context of a case-control study, a statistical test may be performed to test a hypothesis that the methylation profiles of the case subjects differ from the methylation profiles of the control subjects, and the statistical test may perform a comparison between the methylation profiles of the case subjects and the methylation profiles of the control subjects in order to validate or invalidate such hypothesis.

In such context, suitable statistical tests may include Student t-test, Welch test, Wilcoxon-Mann-Whitney test, linear regression, ANOVA, Chi-square test, Fisher's exact test, logistic regression, etc.

In the context of a cohort study, a statistical test may be performed to test a hypothesis that the continuous variable to which the subjects are associated varies with the methylation profiles of the subjects. Suitable statistical tests may include regression tests such as logistic regression tests.

The method then comprises computing, for the CpG sites for which a nominal p-value is available, and preferably for each CpG site represented in the methylation profiles, a transformed p-value taking into account the nominal p-value of the considered CpG site, and the genomic position of this CpG, in order to take into account co-methylation patterns within the DNA, and in particular, correlation of methylation levels within adjacent CpG sites. The transformation of a nominal p-value is thus performed in order to take into account said nominal p-value and the nominal p-values obtained for CpG sites having neighboring DNA positions. For instance, the CpG sites having neighboring DNA positions can be CpG sites belonging to a same CpG-rich region, for instance, but not limitatively, CpG sites belonging to a same CpG island, a same promoter region, or a same gene body.

With reference to figure 1, the transformation may be performed by browsing a methylation profile, where each CpG site is associated to a nominal p-value, with a sliding window of a given width w, such that 2w+1 is the number of CpG sites comprised within the sliding window, and the transformation comprises computing, for the CpG site on which the sliding window is centered, a transformed p-value which is the mean of a log-transform of the nominal p-value of the considered CpG site and of a log-transform of the nominal p-values of the neighboring CpG sites comprised within the sliding window. The transformation thus corresponds to a smoothing of the log-transform of the nominal p-values of the CpG sites. In embodiments, the log-transform of a nominal p-value is $-\log_{10}$(p-value), and the transformation can be denoted with the following equation:

$$Smoothed(Pk, w) = \sum_{n=k-w}^{n=k+w}(-Log10[P - value(Pn)]) \div (2w + 1)$$

Where Smoothed (Pk,w) is the transformed or smoothed p-value of the CpG site of location Pk within the methylation profile, on which the sliding window is centered, w is the width of the sliding window, and P-value is the nominal p-value of a CpG site.

The width of the sliding window is a positive integer strictly greater than 1. The width of the sliding window depends on the resolution of the array of probes used for acquiring the methylation profile. For an array of probes providing methylation levels for approximately 500,000 to 900,000 methylation sites, w can be comprised between 1 and 50, for instance between 1 and 10, for instance between 1 and 5. According to an example, the Infinium Human Methylation 450k Bead Chip array encompasses 482,421 CpG proves and 26,658 CpG islands with an average number of 5.63 CpG probes per CpG island, the window width may be selected between 2 and 5. If an array of probes are capable of providing methylation levels for increased numbers of methylation sites, then the width of the sliding window could be greater in order to take into account co-methylation patterns of CpG sites at a similar scale.

This smoothing transformation may be performed on the nominal p-values output by the statistical test without performing any multiple testing correction of the obtained nominal p-values.

In embodiments, the transformed or smoothed p-values for the plurality of CpG sites may be displayed via a Manhattan plot of said values, in which genomic coordinates of the CpG sites are displayed along the X-axis and the transformed p-values, i.e., the mean of the negative logarithm of the nominal p-values comprised in the sliding window centered on a given CpG site, are displayed along the Y-axis.

With reference to figure 2a is shown the Manhattan plot obtained from an epigenome-wide association study comparing the DNA methylome profiles of 16 subjects with MMACHC promoter epimutation (CpG island Cpg:33) with control subjects. Panel A shows an epi-Manhattan plot with the Y-axis displaying the nominal p-values obtained for the statistical test. Panel B shows the epi-Manhattan plot with the Y-axis displaying the transformed p-values obtained with a sliding window of width 1 (i.e., comprising 3 CpG sites). Panel C shows the epi-Manhattan plot with the Y-axis displaying the transformed p-values obtained with a sliding window of width 2, and panel D with a sliding window of width 3. One can notice that the smoothing of the log-transform of the p-values drastically reduces the amount of noise and allows identifying accurate epigenomic signatures.

In embodiments, the method may further comprise computing a threshold on the transformed p-values and identifying at least one CpG site exhibiting transformed p-values above-said threshold. In embodiments the threshold may be computed according to the standard deviation of the transformed p-values obtained for a plurality, or all, the CpG sites of the methylation profiles.

According to an exemplary embodiment, a threshold may be set as $k^*\sigma^2$, where $\sigma$ is the standard deviation of the transformed p-values obtained for the CpG sites of the methylation profiles, and k is a positive number, for instance, comprised between 1 and 3, for instance, equal to 2. In figure 2, the threshold computed with k=2 is displayed as a line on panels B to D.

In embodiments, the method may further comprise computing a signal-to-noise ratio for a plurality of widths of the sliding window in order to allow determining an appropriate width. Such signal-to-noise ratio can be computed as the ratio $\mu$(signal)/$\sigma$(noise) where $\mu$(signal) corresponds to the mean of the transformed p-values of the CpG probes located in the analyzed to significant locus and $\sigma$(noise) to the standard deviation of the transformed p-values of all the CpG sites located outside the analyzed top significant locus at the genome-wide level, using the same window width value. An example of such signal-to-noise ratio for the same study for which the results are shown in figure 2 is displayed in panel B of figure 3 with different window widths, where 0 corresponds to no smoothing.

In embodiments, the method may further comprise computing a variance reduction between before and after smoothing transformation. An example of such variance reduction is shown in panel A of figure 3 for the same study as the results shown in figure 2.

In embodiments, the method may also comprise computing a variance ratio VAR(Smoothed[Pk,w])(signal)/VAR(Smoothed[Pk,w])(noise) where VAR(Smoothed[Pk,w])(signal) corresponds to the variance computed from the transformed p-values of the CpG probes located in the studied genomic region and VAR(Smoothed[Pk,w])(noise) to the variance calculated from the transformed p-values of all the CpG probes located outside of the studied genomic region at the genome-wide level, using the same window width value. An example of variance ratios for a plurality of window widths is displayed in panel C of figure 3. For the study, which results are shown in figures 2 and 3, the region of interest is the CpG island CpG:33, MMACHC.

**Method for identifying DNA methylation signature**

Aberrant DNA methylation is implicated in many physiological and pathological processes. The identification of DNA methylation signature by performing the method of analysis as described above can serve as measurable indicators of biological conditions, for example, for predicting the presence or severity of a disease state notably at an early stage, the occurrence of disease in longitudinal cohorts, or treatment response and further contribute to the development of clinical treatments and personalized medicine throughout life but also in the identification of DNA samples, body fluid identification and the estimation of ages and phenotypic characteristics. Said pathological state may be disease including as non-limiting examples cancer, obesity, neurological disease, immunological disease, cardiovascular disease, atherosclerosis and addiction. In a particular embodiment, said the pathological state is cancer selected from the group consisting of: breast cancer, ovarian cancer, lung carcinoma, colorectal cancer, prostate cancer, pancreatic cancer, and melanoma.

The present disclosure provides a method for identifying a methylation signature associated with a physiological or pathological state, comprising implementing the method for analyzing DNA methylation profiles as described above wherein said population comprises at least test subjects having said physiological or pathological state, in particular wherein said the pathological state is cancer, preferably selected from the group consisting of: breast cancer, ovarian cancer, lung carcinoma, colorectal cancer, prostate cancer, pancreatic cancer, and melanoma.

In a particular embodiment, the methylation signature associated with a physiological or pathological state is identified by further comparing the DNA methylation profiles of a population comprising test subjects having said physiological or pathological state to a control value.

As used herein, the term "control value" refers to the DNA methylation profile in a biological sample obtained from a general population or from a selected population of subjects. For example, the general population may comprise apparently healthy subjects, such as individuals who have not previously had any sign or symptoms indicating the presence of a physiological or pathological state. The term "healthy subjects" as used herein refers to a population of subjects who do not suffer from any known condition, and in particular, who are not affected with any pathological condition. The control value may be established based upon comparative measurements between subjects not having said physiological or pathological state and subjects having said physiological or pathological state. The control value may alternatively be a control value such as a threshold value, a standard value or a range obtained from other sources than the patient's data. In a particular embodiment, the control value is the DNA methylation profile of a population comprising subject not having said physiological or pathological state.

In another particular embodiment, the methylation signature associated with a physiological or pathological state may correspond to the relationship identified between the methylation level and variations of a physiological or pathological state, in which case the population comprises a plurality of subjects representing said variations of the physiological or pathological state.

The invention will now be exemplified with the following examples, which are not limitative, with reference to the attached drawings in which:

**EXAMPLES**

**MATERIALS AND METHODS**

**1. Example #1: epimutation of the *MMACHC* promoter CpG island (CpG:33)**

*1.1. DNA methylome analysis and quality controls*

The inventors carried out bisulfite conversion of 600 ng of DNA extracted from whole blood using the EZ DNA Methylation kit (Zymo Research, Proteigene, Saint-Marcel, France). The genome-wide profiling of DNA methylome was determined using the Infinium MethylationEPIC BeadChip array (Illumina, Paris, France), according to the manufacturer's instructions. The Infinium MethylationEPIC BeadChip provides coverage of 850,000 CpG probes in enhancer regions, gene bodies, promoters, and CpG islands. The arrays were scanned on an Illumina iScan® system, and raw methylation data were extracted using Illumina's Genome Studio methylation module. For each CpG probe, the methylation level was described as a β value, ranging between 0 (fully unmethylated CpG probe) and 1 (fully methylated CpG probe). Background correction and normalization were implemented using the SWAN method (R Package Minfi) (Aryee, M. J. et al. Bioinformatics (Oxford, England) 30, 1363-1369 (2014)). Probe annotation information, including sequence and chromosome location for the Infinium MethylationEPIC BeadChip array, was retrieved from the Infinium MethylationEPIC v1.0 B5 Manifest File.

*1.2. Description of cases and controls and EWAS design*

The inventors studied 16 subjects with proven *MMACHC* epimutation. All exhibited aberrant methylation on the CpG:33 on the *MMACHC* gene promoter (Chr1: 45,965,587-45,966,049, GRCh37). Of the 16 patients with *MMACHC* epimutation, five were previously reported (Gueant, J. L. et al. Nat Commun 9, 554, (2018)). For the control group, the inventors used in silico data generated using the Infinium Human Methylation 450k BeadChip array from the MARTHA cohort, which included 350 unrelated European-ancestry patients (Dick, K. J. et al. Lancet 383, 1990-1998, (2014)). In the EWAS, the inventors compared the mean β value of each CpG probe between cases and controls using a *t*-test.

**2. Example #2: epimutation of the *MLH1* promoter CpG island (CpG:93)**

The inventors used DNA methylome data generated using the Infinium Human Methylation 450k BeadChip array using blood samples from 12 patients carrying *MLH1* epimutation (CpG: 93, *EPM2AIP1-MLH1*), 61 patients with Lynch syndrome patients, and 41 healthy controls (GSE107351) (Damaso, E. et al. Br J Cancer 119, 978-987, (2018)). In the EWAS, the inventors compared the 12 *MLH1* epimutation carriers (cases) with the 102 remaining patients without *MLH1* epimutation from the series (controls). In the EWAS, the inventors compared the mean β value of each CpG probe between cases and controls using a *t*-test.

**3. Example #3: epigenetic predictors of aging**

The inventors used DNA methylome data generated using the Infinium Human Methylation 450k BeadChip array using blood samples from 656 subjects (426 Caucasians and 230 Hispanics), aged 19 to 101 (GSE40279) (Hannum, G. et al. Mol Cell 49, 359-367, (2013)). The inventors performed an EWAS using linear regression to look for epigenetic predictors associated with age as a continuous dependent variable after adjusting for gender and ethnicity. The inventors selected the CpG probes with a rho Spearman's correlation coefficient ≥0.5 in association with age from the top significant loci. The inventors elaborated the "Polyepigenetic CpG aging score" by calculating the sum of the beta values corresponding to the top CpG probes (n = 15). The inventors assessed the correlation between the "Polyepigenetic CpG aging score" and age using Spearman's rank correlation coefficient. To assess the distribution of the "Polyepigenetic CpG aging score" in an independent dataset, the inventors used DNA methylome data generated using the Infinium Human Methylation 450k from 20 newborns and 20 nonagenarians (ArrayExpress, GEOD-30870) (Heyn, H. et al. Proc Natl Acad Sci U S A 109, 10522-10527, (2012)). The inventors compared the median value of the "Polyepigenetic CpG aging score" between newborns and nonagenarians using the Wilcoxon-Mann-Whitney test.

**4. Quality controls of methylome array data**

The inventors visually inspected the genome-wide distribution of the CpG probes according to their β value. The inventors assessed population stratification according to the whole methylome landscape by performing numeric principal component analysis on normalized beta values of each CpG probe across the methylation array. The top ten principal components (eigenvectors) were calculated with their respective eigenvalue.

**5. Description of the smoothing method for calculating and visualizing data from EWAS results using nominal -Log10(P-values)**

The smoothing method relies on the DNA co-methylation pattern, which is the correlation between adjacent CpG sites. The first description of the co-methylation pattern of CpG sites was reported by Eckhardt *et al.* using bisulfite DNA sequencing to generate high-resolution methylation profiles of human chromosomes 6, 20, and 22, providing a resource of about 1.9 million CpG methylation values derived from 12 different tissues (Eckhardt, F. et al. Nat Genet 38, 1378-1385, (2006)). In this landmark study, over 90% of CpG sites within 50 bases had the same methylation status. Taking into account this assumption, the inventors proposed the conversion of the nominal - Log10(P-value) obtained for each CpG probe at the genome-wide level to smoothed - Log10(P-values) using a pre-specified window width (*w*) value ranging between 1 and 3 (Smoothed[$P_{k,w}$]) (**Figure 1**). The inventors used a symmetric smoothing method so that the Smoothed($P_{k,w}$) of each CpG probe corresponds to the mean of the -Log10(P-values) of the central CpG probe (k) and those located on either side of the central CpG probe within the limit of the pre-defined window width, through a sliding window approach, according to the following formula:

$$Smoothed(Pk, w) = \sum_{n=k-w}^{n=k+w} (-Log10[P - value]) \div (2w + 1)$$

, where k corresponds to the central CpG probe and w to the number of CpG probes on either side of the central CpG probe (window width). The Infinium Human Methylation 450k BeadChip array encompasses 482,421 CpG probes and 26,658 CpG islands with an average number of 5.63 CpG probes per CpG island (Bibikova, M. et al. Genomics 98, 288-295, (2011)). Considering this resolution, the inventors estimated the optimal smoothing width at 2 since this value corresponds to a total number of 5 CpG sites (2w + 1) to calculate the Smoothed($P_{k,w}$) value.

To assess the magnitude of signal enhancement at a given top significant locus, the inventors used several metrics, including the signal-to-noise ratio (SNR), variance reduction, and variance ratio (VR). For a given top significant locus, the signal-to-noise ratio (SNR) was defined as the ratio of the power of a signal (meaningful information at the top locus) and the power of background noise (unwanted signal in the remaining genomic regions at the genome-wide level) according to the following formula (Bushberg, J. T. & Boone, J. M. (Lippincott Williams & Wilkins, 2011)) :

$$Signal - to - noise\ ratio\ (SNR) = \frac{\mu\ (signal)}{\sigma\ (noise)}$$

, where '$\mu$ (signal)' corresponds to the mean of Smoothed($P_{k,w}$) of the CpG probes located in the analyzed top significant locus and '$\sigma$ (noise)' to the standard deviation of Smoothed($P_{k,w}$) of all the CpG probes located outside the analyzed top significant locus at the genome-wide level, using the same window width value (w).

The inventors calculated the variance ratio according to the following formula:

$$Variance\ ratio\ (VR) = \frac{VAR\ (Smoothed[Pk, w])\ (signal)}{VAR\ (Smoothed[Pk, w])\ (noise)}$$

, where 'VAR (Smoothed[$P_{k,w}$]) (signal)' corresponds to the variance calculated from the Smoothed[$P_{k,w}$] of the CpG probes located in the studied genomic region and '*VAR* (Smoothed[$P_{k,w}$]) (noise)' to the variance calculated from the Smoothed[$P_{k,w}$] of all the CpG probes located outside the studied genomic region at the genome-wide level, using the same window width value (w).

The inventors reported the results of epigenome-wide association studies using epi-Manhattan plots for nominal and smoothing transformed -Log10(P-values). In the epi-Manhattan illustrating the smoothing transformation of -Log10(P-values), the inventors used ($2\times\sigma^2$) as a significance threshold value, where $\sigma^2$ corresponds to 'VAR (Smoothed[$Pk,w$]) (noise)', as previously described. The smoothing method and all statistical analyses were performed using the SNP & Variation Suite (v8.8.1; Golden Helix, Inc., Bozeman, MT, USA) and MedCalc, version 19.5.3 (MedCalc Software, Ostend, Belgium).

**RESULTS**

**1. Example #1: epimutation of the *MMACHC* promoter CpG island (CpG:33)**

**DNA methylome analysis and quality controls**

All the DNA methylome profiles of the 16 subjects with an *MMACHC* promoter epimutation (CpG island CpG:33) were of optimal quality and were used for statistical analyses. A total of 449,517 CpG probes were analyzed. As shown in the epi-Manhattan plot, the EWAS using untransformed -Log10(*P*-values) did not retrieve any top significant locus at the genome-wide level **(Figure 2A)**.

After applying a smoothing transformation at the genome-wide level using a window width of 1 (three CpG probes in each window), the inventors observed a top significance at the *MMACHC* promoter CpG island (CpG:33) without any other DNA methylome signature at the genome-wide level. The $SNR_{MMACHC/outside\_MMACHC}$ was increased by -50% (SNR = 16.2) and the $VR_{MMACHC/outside\ MMACHC}$ increased by +95% (VR = 12.7). Using this window width, the median Smoothed($P_{k,1}$) (k: from cg03123370 to cg15896098) of the *MMACHC* promoter CpG island (CpG:33) was 161 (IQR, 129-177) vs. 9 (IQR, 5-15) for the genomic regions outside the CpG:33 of *MMACHC* **(Tables 2-3 and Figure 2B)**.

**Table 2.** Untransformed -Log10(*P*-values) and their smoothed transformation using a window width of 1, 2, and 3 in the CpG island of the *MMACHC* promoter (CpG:33) and outside this genomic region

| | Mini mum | Maxi mum | Me an | Med ian | 25th- 75th | IQR, Varia nce | SD | Varian reducti on (%) | Signal -to- noise Ratio* | Varia nce Ratio † |
|---|---|---|---|---|---|---|---|---|---|---|
| Genomic region: Outside CpG:33 *(MMACHC)* **(449,507** CpG probes) | | | | | | | | | | |
| Untransformed - Log10 *P*-value (window width = 0; 1 CpG probe) | 0 | 311 | 11 | 6 | 2-14 | 225 | 15 | - | - | - |
| Smoothed -Log 10 *P*-value (window width = 1; 3 CpG probes) | 0 | 255 | 11 | 9 | 5-15 | 89 | 9 | -60% | - | - |
| Smoothed -Log 10 *P*-value (window width = 2; 5 CpG probes) | 0 | 212 | 11 | 10 | 6-14 | 45 | 7 | -80% | - | - |
| Smoothed -Log 10 *P*-value (window width = 3; 7 CpG probes) | 0 | 209 | 11 | 10 | 7-14 | 37 | 6 | -84% | - | - |
| **Genomic region: CpG:33 *(MMACHC)* (10 CpG probes)** | | | | | | | | | | |
| Untransformed - LOg10 *P*-value (window width = 0; 1 CpG probe) | 104 | 217 | 163 | 165 | 133-193 | 1467 | 38 | - | 10.9 | 6.5 |
| Smoothed -Log 10 *P*-width = 1; 3 CpG probes) | (window 89 | 197 | 153 161 | | 129- 177 | 1128 34 | | -23% | 16.2 | 12.7 |
| Smoothed -Log 10 *P*-(window width = 2; 5 CpG probes) | 100 | 181 | 139 | 140 | 114- 159 | 810 | 28 | -45% | 20.7 | 18.0 |

(continued)

|  | Mini mum | Maxi mum | Me an | Med ian | 25th- 75th | IQR, Varia nce | SD | Varian reducti on (%) | Signal -to- noise Ratio* | Varia nce Ratio † |
|---|---|---|---|---|---|---|---|---|---|---|
| Genomic region: Outside CpG:33 *(MMACHC)* **(449,507** CpG probes) | | | | | | | | | | |
| Smoothed -Log 10 *P*-(window width = 3; 7 CpG probes) | 103 | 167 | 135 | 131 | 121- 150 | 417 | 20 | -72% | 22.1 | 11.2 |

Note. IQR: interquartile range; SD: standard deviation.

* Signal-to-noise ratio is defined as the ratio of the power of a signal (meaningful information) and the power of background noise (unwanted signal) according to the following formula: SNR = Mean of - Log10(*P*-values) (CpG:33, *MMACHC* gene) / SD of -Log10(*P*-values) (outside CpG:33, *MMACHC* gene).

† Variance-ratio = Variance of -Log10(*P*-values) (CpG:33, *MMACHC* gene) / Variance of -Log10(*P*-values) (outside CpG:33, *MMACHC* gene).

**Table 3. Results of the epigenome-wide association study comparing 11 patients with *MMACHC* epimutation and controls regarding the CpG probes located in the *MMACHC* promoter CpG island (CpG:33)**

| CpG probe | Chr. | Position* | *t*-test *P*-value | n β-value, epi-cblC | **Mean** β-value, Controls | -Log10 *P*-value (Untransformed) (WW = 0) | Smoothed($Pt,1$) (WW = 1) ‡ | Smoothed($P_{k,2}$) (WW = 2) ‡ | Smoothed($P_{k,3}$) (WW = 3) ‡ |
|---|---|---|---|---|---|---|---|---|---|
| cg03123 370† | 1 | 45965 343 | $1.43 \times 10^{-133}$ | 0.51 | 0.11 | 133 | 89 | 100 | 119 |
| cg04700 938† | 1 | 45965 449 | $4.98 \times 10^{-130}$ | 0.44 | 0.06 | 129 | 122 | 108 | 122 |
| cg13848 568 | 1 | 45965 625 | $3.54 \times 10^{-105}$ | 0.36 | 0.04 | 104 | 127 | 131 | 120 |
| cg00081 251 | 1 | 45965 679 | $5.99 \times 10^{-148}$ | 0.51 | 0.07 | 147 | 151 | 149 | 137 |
| cg09323 228 | 1 | 45965 727 | $2.02 \times 10^{-203}$ | 0.57 | 0.05 | 203 | 181 | 156 | 160 |
| cg27393 325 | 1 | 45965 846 | $7.18 \times 10^{-194}$ | 0.56 | 0.02 | 193 | 176 | 169 | 167 |
| cg22536 808 | 1 | 45965 870 | $1.03 \times 10^{-133}$ | 0.40 | 0.00 | 133 | 170 | 181 | 152 |
| cg14836 864 | 1 | 45965 990 | $7.49 \times 10^{-184}$ | 0.49 | 0.03 | 183 | 178 | 160 | 141 |
| cg03108 114 | 1 | 45966 048 | $2.77 \times 10^{-217}$ | 0.59 | 0.02 | 217 | 197 | 131 | 125 |
| cg15896 098 | 1 | 45966 115 | $2.11 \times 10^{-192}$ | 0.56 | 0.03 | 192 | 136 | 105 | 103 |

Note. Chr.: chromosome; WW: window width.

* Genomic positions according to GRCh37 (hgl9).

† The two CpG probes, cg03123370 and cg04700938, were in the vicinity of the CpG island:33 and were included in the analysis of the *MMACHC* locus.

‡ The median and IQR -Log10(*P*-values) much lower in the CpG probes outside the CpG:33, *MMACHC* gene were equal to 6 (IQR, 2-14), 9 (5-15), 10 (6-14), and 10 (7-14) for the untransformed -Log10(*P*-values) and smoothed -Log10(*P*-values) using a window width of 1, 2, or 3, respectively.

The increase in VR$_{MMACHC/outside\_MMACHC}$ was related to a -61% decrease in noise variance (outside CpG:33, *MMACHC)* that was concomitant with a decrease but to a much lesser extent (-23%) of the signal variance (CpG:33, *MMACHC)* (**Table 2** and **Figure 3**).

The smoothing transformation using a window width of 2 (five CpG probes in each window) achieved a +90% increase of the SNR$_{MMACNC/outside\_MMACHC}$ (20.7) and a +176% increase in the *VR*$_{MMACHC/outside\_MMACHC}$ (18.8). Using this window width, the median Smoothed($P_{k,2}$) of the *MMACHC* promoter CpG island (CpG:33) was 140 (IQR, 114-159) vs. 10 (IQR, 6-14) for the genomic regions outside the CpG:33 of *MMACHC* (**Tables 2-3** and **Figure 2C**). Using the window width of 2, the decrease in noise variance was -80%, while that of the signal variance (CpG:33, *MMACHC)* was -45% (**Table 2** and **Figure 3**).

The smoothing transformation using a window width of 3 (seven CpG probes in each window) provided a 103% increase of the SNR$_{MMACNC/outside\_MMACHC}$ (22.1) and a +73% increase in the *VR*$_{MMACHC/outside\_MMACHC}$ (11.2) (**Table 2** and **Figure 2D**). Using this window width, the median Smoothed($P_{k,3}$) of the *MMACHC* promoter CpG island (CpG:33) was 131 (IQR, 121-150) vs. 10 (IQR, 7-14) for the genomic regions outside the CpG:33 of *MMACHC* (**Tables 2-3** and **Figure 2D**). Using the window width of 3, the decrease in noise variance was -84%, while that of the signal variance (CpG:33, *MMACHC)* was -72% (**Table 2** and **Figure 3**).

### 2. Example #2: epimutation of the *MLH1* promoter CpG island (CpG:93)

The EWAS using untransformed -Log10(*P*-values) retrieved a significant association in the *EPM2AIP1-MLH1* CpG island (CpG:93, gene promoter) (**Figure 4A**) with a hemimethylated profile among patients harboring the CpG:93 epimutation when compared to controls who had a fully unmethylated CpG:93 (**Figure 4B**). The smoothing transformation using a window width of 3 maintained the top significance at CpG:93, reduced the noise variance in the remaining genomic regions, and did not retrieve any other DNA methylome signature at the genome-wide level (**Figure 4A**).

### 3. Example #3: epigenetic predictors of aging

Using linear regression EWAS, the inventors looked for epigenetic predictors associated with age as a continuous dependent variable after adjusting for gender and ethnicity. The EWAS using untransformed -Log10(*P*-values) found no clear association at the genome-wide level (**Figure 5A**). After applying the smoothing transformation and a window width of 3, the inventors retrieved a top significance at four loci: *ZYG11A* (CpG:112, gene promoter), *FHL2* (CpG:81, gene promoter), *ELOVL2/ELOVL2-AS1* (CpG: 141 bidirectional promoter of the head-to-head sense-antisense gene pair), and *KLF14* (CpG:156, gene promoter) (**Figure 5B**). Fifteen CpG probes from the four top significant loci were highly and positively correlated with age (**Table 4**).

| CpG probe | Chrom. | Position | Gene | CpG island | rho | Two-sided P-value |
|---|---|---|---|---|---|---|
| cg16015712 | 1 | 53308597 | *ZYG11A* | CpG: 112 | 0.66 | 2.15E-84 |

| cg06335143 | 1 | 53308654 | *ZYG11A* | CpG: 112 | 0.55 | 1.65E-52 |
|---|---|---|---|---|---|---|
| cg06784991 | 1 | 53308768 | *ZYG11A* | CpG: 112 | 0.72 | 2.16E-105 |
| cg24466241 | 1 | 53308908 | *ZYG11A* | CpG: 112 | 0.63 | 1.09E-73 |
| cg06639320 | 2 | 106015739 | *FHL2* | CpG: 81 | 0.76 | 1.54E-123 |
| cg22454769 | 2 | 106015767 | *FHL2* | CpG: 81 | 0.73 | 1.28E-109 |
| cg24079702 | 2 | 106015771 | *FHL2* | CpG: 81 | 0.72 | 1.03E-104 |
| cg16867657 | 6 | 11044877 | *ELOVL2 \| ELOVL2-AS1* | CpG: 141 | 0.86 | 3.37E-196 |
| cg24724428 | 6 | 11044888 | *ELOVL2 \| ELOVL2-AS1* | CpG: 141 | 0.77 | 6.43E-130 |
| cg21572722 | 6 | 11044894 | *ELOVL2 \| ELOVL2-AS1* | CpG: 141 | 0.72 | 4.14E-105 |
| cg14361627 | 7 | 130419116 | *KLF14* | CpG: 156 | 0.62 | 3.05E-70 |
| cg08097417 | 7 | 130419133 | *KLF14* | CpG: 156 | 0.61 | 1.75E-67 |
| cg09499629 | 7 | 130419136 | *KLF14* | CpG: 156 | 0.48 | 1.09E-39 |
| cg07955995 | 7 | 130419159 | *KLF14* | CpG: 156 | 0.63 | 8.15E-73 |
| cg22285878 | 7 | 130419173 | *KLF14* | CpG: 156 | 0.62 | 1.40E-69 |

**Table 4.**

The 'Polyepigenetic CpG aging score' based on the 15 top CpG was highly and positively correlated with age (Spearman's rho = 0.89; 95% CI, 0.88-0.90; *P* = 3.00x10$^{-219}$) (**Figure 5C**). Notably, on the four top loci retrieved in the present analysis, two were highlighted in the original published study *(ELOVL2, KLF14),* and two remaining loci have been very well documented in later studies as being involved in age-related epigenomic signatures (Naue, J. et al. Forensic Sci Int Genet 31, 19-28, (2017); Florath, I., et al. Hum Mol Genet 23, 1186-1201, (2014); Tan, Q. et al. Int J Epidemiol 45, 1146-1158, (2016) ; Jung, S. E. et al. Forensic Sci Int Genet 38, 1-8, (2019) ; Bacalini, M. G. et al. J Gerontol A Biol Sci Med Sci 72, 1015-1023, (2017); Garagnani, P. et al. Aging Cell 11, 1132-1134, (2012); Kananen, L. et al. BMC Genomics 17, 103, (2016)). The inventors performed a sensitivity analysis on independent sub-samples by randomly selecting five subgroups, each of them representing 20% of the total population (**Table 5**).

| | Bootstrap group 1 | Bootstrap group 2 | Bootstrap group 3 | Bootstrap group 4 | Bootstrap group 5 |
|---|---|---|---|---|---|
| N | 132 | 131 | 131 | 131 | 131 |
| Minimal, age | 21 | 23 | 19 | 22 | 27 |
| Maximal, age | 92 | 101 | 89 | 96 | 93 |
| Median, age | 62 | 65 | 66 | 65 | 64 |
| 25$^{th}$ – 75$^{th}$, IQR | 51–74 | 53–77 | 58–75 | 57–76 | 53–73 |

**Table 5. Distribution of age in the five randomly selected subgroups**

N: number of subjects; IQR: interquartile range.

Using a Smoothed($P_{k,3}$) threshold of 10, all the five sensitivity analyses confirmed the *FHL2, ELOVL2/ELOVL2-AS1,* and *KLF14* loci (**Figure 6**). Using the same Smoothed($P_{k,3}$) threshold, the *ZYGA11* locus was confirmed in three EWAS (**Figure 6**). In the replication EWAS, the Polyepigenetic CpG aging score differed significantly between newborns and nonagenarians with a median score of 2.00 (IQR, 1.89-2.08) and 5.60 (IQR, 5.52-5.72), respectively (P = 3.40×10$^{-8}$) (**Figure 7**).

**CONCLUSION**

The inventors showed that by applying the smoothing method, they optimized the signal-to-noise ratio, which allowed highlighting potentially relevant epigenomic signatures. The optimization of the signal-to-noise ratio resulted from a downward shift (shift to the left) of the distribution of the -Log10(*P*-values) at the genome-wide level, allowing background noise to be reduced while maintaining the strength of the signal **(Figure 8)**. Interestingly, the signal-to-noise ratio approach has been tested to uncover de novo locus-disease discoveries in genome-wide association studies. It was suggested as a potential alternative to multiple testing correction approaches that could result in an over-conservative correction and a reduced power to detect true associations.

The inventors demonstrated that the smoothing method effectively uncovers epigenomic signatures in monogenic epigenetic disorders with small sample size datasets but also in large sample size EWAS on complex phenotypes. Importantly, in the case of large datasets, the smoothing method provided similar results even after applying an 80% reduction of the original sample size. The present results suggest revisiting EWAS by applying the smoothing method to already available datasets to re-analyze and potentially identify highly accurate epigenomic signatures that could translate into biologically meaningful results.

**Claims**

1.  A computer-implemented method for analyzing methylation profiles of DNA samples of a population, where each methylation profile comprises a methylation level of cytosine of a plurality of CpG sites of determined genomic DNA positions, the method comprising:

    i) performing a statistical test on said methylation profiles of the population, and obtaining a nominal p-value for a plurality of said CpG sites,
    ii) for at least one CpG site, computing a transformed p-value taking into account the nominal p-value for the considered CpG site and its genomic position.

2.  The method of claim 1, wherein the computation of the transformed p-values takes into account the nominal p-value obtained for the considered CpG site and the nominal p-values obtained for CpG sites having neighboring DNA positions.

3.  The method of claim 1 or 2, wherein the computation of the transformed p-values takes into account the nominal p-value obtained for the considered CpG site and the nominal p-values obtained for CpG sites belonging to respectively a same CpG-rich region to which belongs the considered CpG site.

4.  The method of any of claims 1 to 3, wherein computing a transformed p-value comprises computing, for a given CpG site, a mean of log-transformed nominal p-values of the CpG sites comprised in a sliding window centered on the given CpG site and including 2w+1 CpG sites where w is a positive integer designating the width of the sliding window.

5.  The method according to claim 4, wherein computing a transformed p-value comprises computing, for a given CpG site a value defined by the following equation:

$$Smoothed(Pk, w) = \sum_{n=k-w}^{n=k+w} (-Log10[P-value(Pn)]) \div (2w+1)$$

    where Smoothed(Pk,w) is the transformed p-value for the CpG site of location Pk within the methylation profile, w is the width of the sliding window, and P-value(Pn) is the nominal p-value of the CpG site of location Pn.

6.  The method according to any of the preceding claims, further comprising computing a threshold on the transformed p-values and identifying at least one CpG site exhibiting a transformed p-value higher than said threshold.

7.  The method according to any of the preceding claims, further comprising displaying a Manhattan plot of the transformed p-values.

8. The method according to any of the preceding claims wherein said methylation level is the ratio of methylated cytosine and the sum of methylated and unmethylated cytosines.

9. The method according to any of the preceding claims, comprising prior to step i) the step of determining methylation level in said DNA samples of a population.

10. The method of claim 9 wherein the step of determining said methylation level comprises a bisulfite pre-treatment of the DNA samples which converts unmethylated cytosines into uracils.

11. The method according to claim 9 or 10 wherein the step of determining said methylation level comprises applying DNA samples on microarray chips of probes.

12. A method for identifying a methylation signature associated with a physiological or pathological state comprising implementing the method according to any one of claims 1 to 11 wherein said population comprises at least test subjects having said physiological or pathological state.

13. The method according to claim 12 wherein the population further comprises control subjects not having said pathological or physiological state.

# No smoothing (radius = 0)

Unsmoothed -Log10(P-value)

# Smoothing radius = $r$

$$k\text{-}3 \quad k\text{-}2 \quad k\text{-}1 \quad k \quad k\text{+}1 \quad k\text{+}2 \quad k\text{+}3$$

$$\text{Smoothed}(Pk, w) = \sum_{n=k-w}^{n=k+w} (-Log10[P-value]) \div (2w + 1)$$

FIG. 1

FIG. 2

FIG. 2

FIG. 3

# A *MLH1* epimutation vs. controls: genome-wide comparison

T Test –log10 *P*-value (*MLH1* epimutation vs. controls) (No smoothing)

*EPM2AIP1–MLH1 locus*
*(CpG: 93)*

T Test –log10 *P*-value (*MLH1* epimutation vs. controls) (Smoothed, radius = 3)

*EPM2AIP1–MLH1 locus*
*(CpG: 93)*

**FIG. 4**

**B** *MLH1* epimutation vs. controls : *EPM2AIP1–MLH1* locus (CpG: 93)

T Test –log10 *P*-value (*MLH1* epimutation vs. controls) (No smoothing)

T Test –log10 *P*-value (*MLH1* epimutation vs. controls) (Smoothed, radius = 3)

**FIG. 4 (CONTINUED)**

B

Average beta values: *MLH1* epimutation vs. controls

CpG Islands 2009-03-08, UCSC

RefSeq Genes 105 Interim v3, NCBI

FIG. 4 (CONTINUED)

**A Linear regression (age), No smoothing**
−log10 FvR Model P (No smoothing) (age as continuous variable, adjusted for gender and ethnicity)

**B Linear regression (age), Smoothed**
−log10 FvR Model P (Smoothed, radius = 3) (age as continuous variable, adjusted for gender and ethnicity)

ZYG11A
FHL2
ELOVL2 / ELOVL2-AS1
KLF14

**FIG. 5**

C  Polyepigenetic CpG aging score

FIG. 5

FIG. 6

FIG. 7

**FIG. 8**

Europäisches
Patentamt

European
Patent Office

Office européen
des brevets

# EUROPEAN SEARCH REPORT

**Application Number**

EP 21 30 6458

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG ZHEN ET AL: "swDMR: A Sliding Window Approach to Identify Differentially Methylated Regions Based on Whole Genome Bisulfite Sequencing", PLOS ONE, vol. 10, no. 7, 15 July 2015 (2015-07-15), page e0132866, XP055907217, DOI: 10.1371/journal.pone.0132866 Retrieved from the Internet: URL:https://www.ncbi.nlm.nih.gov/pmc/articles/PMC4503785/pdf/pone.0132866.pdf> * the whole document * * in particular * * page 2 - page 4; figure 2 * ----- | 1-13 | INV. G16B20/40 G16B40/10 G16B20/30 |
| X | ZACHARYF GERRING ET AL: "Genome-wide DNA methylation profiling in whole blood reveals epigenetic signatures associated with migraine", BMC GENOMICS, BIOMED CENTRAL LTD, LONDON, UK, vol. 19, no. 1, 22 January 2018 (2018-01-22), pages 1-10, XP021252740, DOI: 10.1186/S12864-018-4450-2 * the whole document * * in particular * * "methods", "results" sections * ----- -/-- | 1-13 | TECHNICAL FIELDS SEARCHED (IPC) G16B |

The present search report has been drawn up for all claims

| Place of search | Date of completion of the search | Examiner |
|---|---|---|
| Munich | 30 March 2022 | Rákossy, Z |

Europäisches
Patentamt

European
Patent Office

Office européen
des brevets

# EUROPEAN SEARCH REPORT

Application Number

EP 21 30 6458

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LUZ D OROZCO ET AL: "Epigenome-wide association in adipose tissue from the METSIM cohort", HUMAN MOLECULAR GENETICS, vol. 27, no. 10, 16 March 2018 (2018-03-16), pages 1830-1846, XP055642135, GB ISSN: 0964-6906, DOI: 10.1093/hmg/ddy093 * the whole document * * in particular * * abstract * * page 1843 * | 1-13 | |
| X | US 2019/323090 A1 (WIDSCHWENDTER MARTIN [AT] ET AL) 24 October 2019 (2019-10-24) * the whole document * * in particular * * paragraph [0314] - paragraph [0315] * | 1-13 | |
| X | HACKENBERG MICHAEL ET AL: "Prediction of CpG-island function: CpG clustering vs. sliding-window methods", BMC GENOMICS, BIOMED CENTRAL LTD, LONDON, UK, vol. 11, no. 1, 26 May 2010 (2010-05-26), page 327, XP021072645, ISSN: 1471-2164, DOI: 10.1186/1471-2164-11-327 * the whole document * * in particular * * "results and discussion" section * | 1-13 | TECHNICAL FIELDS SEARCHED (IPC) |

The present search report has been drawn up for all claims

| Place of search | Date of completion of the search | Examiner |
|---|---|---|
| Munich | 30 March 2022 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6458

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2022

| Patent document cited in search report | | Publication date | Patent family member(s) | | Publication date |
|---|---|---|---|---|---|
| US 2019323090 | A1 | 24-10-2019 | AU 2017375038 | A1 | 04-07-2019 |
| | | | AU 2017378196 | A1 | 04-07-2019 |
| | | | CA 3047202 | A1 | 21-06-2018 |
| | | | CA 3047208 | A1 | 21-06-2018 |
| | | | EP 3336197 | A1 | 20-06-2018 |
| | | | EP 3555308 | A1 | 23-10-2019 |
| | | | EP 3555309 | A1 | 23-10-2019 |
| | | | US 2019323090 | A1 | 24-10-2019 |
| | | | US 2019330703 | A1 | 31-10-2019 |
| | | | WO 2018109212 | A1 | 21-06-2018 |
| | | | WO 2018109217 | A1 | 21-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02086163 A1
- US 5786146 A
- US 6265171 B
- US 6251594 B
- US 6331393 B
- US 7611869 B
- WO 2004051224 A

### Non-patent literature cited in the description

- **MICHELS, K. B. et al.** *Nat Methods,* 2013, vol. 10, 949-955
- **VAN ITERSON, M. et al.** *Genome Biol,* 2017, vol. 18, 19
- **MALLIK, S. et al.** *Brief Bioinform,* 2019, vol. 20, 2224-2235
- **PEDERSEN, B. S. et al.** *Bioinformatics,* 2012, vol. 28, 2986-2988
- **JAFFE, A. E. et al.** *Int J Epidemiol,* 2012, vol. 41, 200-209
- **ZOU, J. et al.** *Nat Methods,* 2014, vol. 11, 309-311
- **LI, H., HONG et al.** *Gene,* 2015, vol. 555, 203-207
- **LEHNE, B et al.** *Genome Biol,* 2015, vol. 16, 37
- **FAN, S ; CHI, W.** *Brief Funct Genomics,* 2016, vol. 15, 432-442
- **CHEN, J. et al.** *BMC Genomics,* 2017, vol. 18, 413
- **HEISS, J. A. et al.** *Epigenomics,* 2017, vol. 9, 13-20
- **RAHMANI, E. et al.** *Genome Biol,* 2018, vol. 19, 141
- **ZHENG, S. C. et al.** *Nat Methods,* 2018, vol. 15, 1059-1066
- **SAFFARI, A. et al.** *Genet Epidemiol,* 2018, vol. 42, 20-33
- **SRIVASTAVA, A. et al.** *Bioinformatics,* 2019, vol. 20, 253
- **BIBIKOVA, M. et al.** *Genome Res,* 2006, vol. 16, 1075-1083
- **HEBESTREIT, K. ; DUGAS, M. ; KLEIN, H. U.** *Bioinformatics,* 2013, vol. 29, 1647-1653
- **XIE, H. et al.** *Nucleic Acids Res,* 2011, vol. 39, 4099-4108
- **ANGENENT, S. ; PICHON, E. ; TANNENBAUM, A.** *Bulletin of the American mathematical society,* 2006, vol. 43, 365-396
- **O'HAVER, T. ; BEGLEY.** *Analytical Chemistry,* 1981, vol. 53, 1876-1878
- **CZANNER, G. et al.** *Proc Natl Acad Sci U S A,* 2015, vol. 112, 7141-7146
- **QI, J. et al.** *Nat Commun,* 2018, vol. 9, 1848
- **ECKHARDT, F. et al.** *Nat Genet,* 2006, vol. 38, 1378-1385
- **BELL, J. T. et al.** *Genome Biol,* 2011, vol. 12, R10
- **BIBIKOVA, M. et al.** *Genomics,* 2011, vol. 98, 288-295
- **HUANG et al.** *Human Mol. Genet.,* 1999, vol. 8, 459-70
- **PLASS et al.** *Genomics,* 1999, vol. 58, 254-62
- **GONZALGO et al.** *Cancer Res.,* 1997, vol. 57, 594-599
- **TOYOTA et al.** *Cancer Res.,* vol. 59, 2307-2312
- **CLARK et al.** *Nucleic Acids Res.,* 1994, vol. 22, 2990-2997
- **FROMMER et al.** *PNAS USA,* 1992, vol. 89, 1827-1831
- **HERMAN et al.** *Proc. Natl. Acad. Sci. USA,* 1996, vol. 93, 9821-9826
- **GONZALGO ; JONES.** *Nucleic Acids Res.,* 1997, vol. 25, 2529-253 1
- **EADS et al.** *Cancer Res.,* 1999, vol. 59, 2302-2306
- **HEID et al.** *Genome Res,* 1996, vol. 6, 986-994
- **ARYEE, M. J. et al.** *Bioinformatics (Oxford, England),* 2014, vol. 30, 1363-1369
- **GUEANT, J. L. et al.** *Nat Commun,* 2018, vol. 9, 554
- **DICK, K. J. et al.** *Lancet,* 2014, vol. 383, 1990-1998
- **DAMASO, E. et al.** *Br J Cancer,* 2018, vol. 119, 978-987
- **HANNUM, G. et al.** *Mol Cell,* 2013, vol. 49, 359-367
- **HEYN, H. et al.** *Proc Natl Acad Sci U S A,* 2012, vol. 109, 10522-10527

- **NAUE, J. et al.** *Forensic Sci Int Genet,* 2017, vol. 31, 19-28
- **FLORATH, I. et al.** *Hum Mol Genet,* 2014, vol. 23, 1186-1201
- **TAN, Q. et al.** *Int J Epidemiol,* 2016, vol. 45, 1146-1158
- **JUNG, S. E. et al.** *Forensic Sci Int Genet,* 2019, vol. 38, 1-8
- **BACALINI, M. G. et al.** *J Gerontol A Biol Sci Med Sci,* 2017, vol. 72, 1015-1023
- **GARAGNANI, P. et al.** *Aging Cell,* 2012, vol. 11, 1132-1134
- **KANANEN, L. et al.** *BMC Genomics,* 2016, vol. 17, 103